# EUROPÄISCHE PATENTSCHRIFT

(11) **EP 1 184 040 B1**
(45) Veröffentlichungstag und Bekanntmachung des Hinweises auf die Patenterteilung: **29.10.2003**
(21) Anmeldenummer: 01119479.2
(22) Anmeldetag: 14.08.2001
(51) Int. Cl.: A61L 27/38, A61L 27/60

(54) **Hautmatrix zur Abdeckung und Regenerierung verletzter Hautpartien sowie Verfahren zu ihrer Herstellung**
Skin matrix for covering and regeneration of injured skin parts and process for making the same
Matrice de peau pour la couverture et regénération de parties de peau lésées et son procédé de fabrication

(30) Priorität: 23.08.2000 DE 10041468
(43) Veröffentlichungstag der Anmeldung: 06.03.2002
(73) Patentinhaber: Surface Care GmbH, 75031 Eppingen (DE)
(72) Erfinder: Lang, Ekkehard, 35796 Weinbach (DE); Schneider, Henning, 75031 Eppingen (DE)
(74) Vertreter: Grussdorf, Jürgen, Dr.

(56) Entgegenhaltungen:
- WO-A-00/32252
- WO-A-96/33750
- WO-A-99/00152
- KREIS R W ET AL: "Expansion techniques for skin grafts: Comparison between mesh and Meek island (Sandwich-) grafts." BURNS, Bd. 20, Nr. SUPPL. 1, 1994, Seiten S39-S42, XP001050485 ISSN: 0305-4179
- RUPP G ET AL.: "Fixierung von Mesh-Grafts mit der Fibrinklebetechnik" LANGENBECKS ARCHIV FUER CHIRURGIE, Bd. 358, Nr. 1, 1982, Seite 563 XP001050555 ISSN: 0023-8236

## Beschreibung

Gegenstand der vorliegenden Erfindung ist eine neue Hautmatrix zur Abdeckung und Regenerierung verletzter Hautpartien sowie Verfahren zu ihrer Herstellung.

Der großflächige Ersatz von Oberhaut (Epidermis) und der darunterliegenden Lederhaut (Dermis) ist außer bei seltenen Abschürfungen oder chirurgischen Eingriffen überwiegend bei solchen Verbrennungen, Verätzungen und chronischen Wunden notwendig, die die Haut in ihrer gesamten Dicke beschädigen.

### Stand der klinischen Behandlung von Verbrennungswunden

Der frühe und permanente Verschluss von Verbrennungswunden stellt nach wie vor ein zentrales Problem bei Patienten mit ausgedehnten Verbrennungen dar. Mit den Verbesserungen in der intensivmedizinischen Behandlung und bei frühzeitiger Nekrektomie (GERMANN et al., Fremdhauttransplantation bei Schwerverbrannten, Chirurg 66 (1995) 260-279) können auch Patienten mit ausgedehnten tiefen Verbrennungswunden überleben. Der möglichst frühzeitige definitive Verschluss großflächiger Wunden vermeidet Flüssigkeitsverluste und Infektionen, die Hauptursachen für die ansonsten hohe Mortalität. Wenn nicht genügend körpereigenes Hautmaterial zur Verfügung steht, entsteht der Bedarf nach biokompatiblen Haut-äquivalenten zur dauerhaften Deckung der Brandwunden.

Die vitale Bedrohung durch die verzögerte Wunddeckung nach großflächigem thermischen Hautverlust führte zur Entwicklung der autologen Keratinozytentransplantation mit konfluenten Keratinozytensheets (GALLICO et al.: Permanent coverage of large burn wounds with autologous cultured human epithelium. N Engl J Med 311 (1984) 448-451). Mit dieser medizinisch anerkannten Methode werden seit 1986 lebenserhaltende permanente Wunddeckungen durchgeführt (seit 1990 an ca. 150 schwerverbrannten Patienten in Deutschland und an mehr als 2000 Patienten weltweit). Auf nichtinfizierten, gut vaskularisierten Wundbetten adhärieren die Kulturzellen, proliferieren und differenzieren innerhalb einer Woche zu einem epidermalen Abschlussgewebe. Langzeitstudien (COMPTON et al.: Skin regenerated from cultured epithelial autografts on full-thickness burn wounds from 6 days to 5 years after grafting. Lab Invest 60 (1989) 600-612) belegen die schnelle physiologische Anbindung der kultivierten Epidermis an das Wundbett, ihre volle Ausreifung erfordert dagegen mehr als ein Jahr. Zur Neubildung des Bindegewebes, einer vaskularisierten Dermis mit retikulären Anteilen und elastischen Eigenschaften, benötigt der Körper dagegen vier bis fünf Jahre. Die regenerierte Haut bleibt dabei ohne Schweißdrüsen, Haarfollikel und Talgdrüsen.

Die aus Hautbiopsien gezüchteten autologen Keratinozyten ermöglichen als physiologischer Hautersatz einen permanenten Wundverschluss und das Überleben von Schwerbrandverletzten können jedoch aufgrund des Fehlens dermaler Komponenten zu einem reduzierten Gesamt-"Take" führen. Der erreichte Hautverschluss ist zunächst mechanisch fragil und infektanfällig und kann langfristig granulationsbedingte Narbenbildungen bzw. Kontrakturneigungen nicht sicher verhindern. (HICKERSON: Technical advances in the utilization of cultured epidermal autografts: Dermal augmentation for wound bed preparation. American Bum Association,Twenty-nineth Annual Meeting Postgraduate Course; New York City; Proc Amer Bum Assoc. (1997)).

Die Chirurgie fordert deshalb seit langem die Integration dermaler Äquivalente in die Transplantate, bzw. die gleichzeitige Applikation dermaler Strukturelemente, um einen frühzeitig belastbaren, dauerhaften Hautersatz zu gewährleisten. Seit 1997 werden sogenannte Komposit-Transplantate, also Transplantate, die im Labor aus der Kombination autologer Epidermiszellen mit allogener Dermis oder artifiziellen Matrizes hergestellt werden, als optimale Möglichkeit für einen permanenten Hautersatz angesehen. Gefordert werden für replantierbare Abschlussgewebe neben anderen Kriterien wie der Unterstützung lokaler Abwehrmechanismen und Wundheilung verbesserte elastische Eigenschaften, ein Wachstumspotential möglichst gleich dem der patienteneigenen Haut und eine langfristig bessere mechanische und ästhetische Qualität.

Werden in Zellkultur vermehrte Epidermiszellen auf oder kombiniert mit einer Matrix transplantiert, können "Take"-Raten bis zu 100% erreicht werden (COMPTON: Cultured epithelial autografts for burn wound resurfacing: Review of observations from a 13-year biopsy study, American Bum Association,Twenty-nineth Annual Meeting Postgraduate Course; New York City; Proc Amer Burn Assoc. (1997)). Auf der Grundlage von Versuchen an athymischen Mäusen wurden diverse Kombinationen aus Zellen epidermaler (Keratinozyten) und dermaler (Fibroblasten) Herkunft mit dezellulierter, avitaler allogener Dermis oder dermalen Analoga entwickelt und auch am Menschen angewendet.

Ergebnisse mit diesen Transplantaten aus Keratinozytenkulturen und humaner azellulärer Dermis zeigten eine Verbesserung der klinischen Gesamtresultate (MEDALIE et al.: Differences in dermal analogs influence subsequent pigmentation, epidermal differentiation, basement membrane, and rete ridge formation of transplanted composite skin grafts, Transplantation 64(3) (1997) 454-465), selbst auf epifaszialen Wunden (BOYCE: Epidermal / dermal co-cultures - current status, American Burn Association, Twenty-nineth Annual Meeting Postgraduate Course; New York City; Proc Amer Burn Assoc. (1997)). Solche Komposittransplantate gelten also als bedeutender Fortschritt auf dem Weg zur Heilung tiefer Verbrennungswunden.

Aus Kreis, R. W. et. al., Burns (1994) 20, Seite S 39-S 42 ist bekannt, Hautmatrizen aus einem Netz aus gemeshter und nach der Meek-Technik weitlumig expandierter autologer oder allogener humaner Haut herzustellen und als Wundabdeckung auch von tiefen Verbrennungen zu verwenden.

Aus WO 99-00152 ist bekannt, ein allogenes oder xenogenes Gewebematerial von den enthaltenen antigen reaktiven Zellen zu befreien und in die erhaltene aufgelockerte Collagenmatrix autologe Zellen des Empfängers oder angepasste Zellen einzusiedeln und als Transplantat zu verwenden.

Aus Rupp G. et. al., Langenbecks Archiv für Chirurgie (1982) 358 (1) Seite 563, ins bekannt, Haut oder Spalthaut mit Fibrinklebern auf Hautverletzungen zu fixieren und gegebenenfalls noch mit einer Schicht aus Fibrinepithelbrei zu überdecken.

Grundsätzlich sind zwei Ansätze bei der Verwendung von Komposithaut zu unterscheiden:

### 1.) Zwei-Schritt-Techniken

Hierbei erfolgt nach vollständiger Wundexzision die Applikation eines für den permanenten Verbleib bestimmten dermalen Ersatzes. Dieser wird eingeheilt und erst nach seiner Vaskularisierung auf der Wunde um eine aus dem Labor stammende permanent verbleibende patienteneigene Neoepidermis komplettiert. Die Wunddeckung mit kryokonservierter Haut, gefolgt von sekundärer Transplantation mit kultivierten, autologen Keratinozyten nach Rheinwald/ Green, ist hierfür ein Beispiel.

### 2.) Ein-Schritt-Techniken

Hierbei erfolgt nach Wundexzision die simultane Applikation von dermalem und epidermalem Ersatzgewebe. Als Beispiele für dauerhafte Komposithauttransplantate können Versuche der Kokultivierung von Keratinozyten und Fibroblasten (LifeSKIN®, Culture Technology, Inc.), Kollagen-Glykosaminglykan-Gel mit autologen Fibroblasten und Keratinozyten oder von Polyethylen-Oxid/Polybutylen-Terephthalaten mit autologen Fibroblasten und Keratinozyten (PolyActive®) gelten. Keines dieser Produkte wurde jedoch bis dato reproduzierbar realisiert oder ist in absehbarer Zeit hierzulande für eine großflächige Anwendung verfügbar.

Die wesentlichen Unterschiede zwischen der Ein-Schritt- und Zwei-Schritt-Technik, liegen im Vaskularisierungsgrad des transplantierten Wundgrundes bzw. der dermo-epidermalen Verbindung an zwischen Neodermis und Neoepidermis.

Bei der Zwei-Schritt-Technik besteht zum Zeitpunkt der Transplantation keine Verbindung von dermalem und neoepidermalem Ersatzgewebe, das dermale Ersatzgewebe auf der Wunde ist jedoch vollständig vaskularisiert. Bei der Ein-Schritt-Technik wird die Verbindung zwischen dermalem und epidermalem Ersatzgewebe bereits in vitro vor der Transplantation erzeugt, wohingegen der dermale Ersatz zum Zeitpunkt der Transplantation avaskulär ist.

Resümierend muss daher festgestellt werden, dass kein bisheriges Hautersatzmaterial humanen Ursprungs ein komplettes hautähnliches und dauerhaftes Integument liefert, wie es eigentlich gewünscht ist. Das Fehlen der Gefäßversorgung macht die Transplantate anfällig gegen Infektionen. Die mangelnde Stoffversorgung der Zellen kann zu verlangsamter Einheilung bis hin zum Transplantatverlust führen.

Die Verwendung von Kompositen in der Klinik ist derzeit noch als hoch experimentell einzustufen. Um den histologischen und physiologischen Gegebenheiten weitestgehend zu entsprechen, erfordern anheftungsabhängige Zellen zur Herstellung eines transplantierbaren Hautäquivalentes die Kombination mit einer biologischen oder biokompatiblen Matrix.

Die hierbei verwendeten biokompatiblen und/oder biodegradierbaren Materialien werden vom Körper angenommen, im Laufe der Zeit abgebaut und durch körpereigenes Material ersetzt. Aktuelle Entwicklungen stellen eine Vielzahl von biokompatiblen, dreidimensionalen Kulturmatrizes zur Verfügung, deren Funktionalität teilweise bereits im experimentellen Maßstab gezeigt werden konnte (BOYCE: Epidermal / dermal co-cultures - current status, American Bum Association, Twenty-nineth Annual Meeting Postgraduate Course; New York City; Proc Amer Burn Assoc. (1997)).

Die Verträglichkeit einiger Matrizes, für die teilweise Zulassungen als Medizinprodukte vorliegen, konnte mit kultivierten Zellen im experimentellen Maßstab gezeigt werden.
1) Polylactide; z.B. Ethisorb® Ethicon, V7-2 iTV® Denkendorf
2) Polyesterurethan; z.B. Degrapol®
3) Hyaluronsäureester; z.B. HYAFF®, Smith & Nephew, Laserskin® Fidia
4) Mikrosphären-Techniken Kollagen/Dextran-Mikrospären, z.B. Roche
5) Kollagenderivate bzw. Kollagen-Glykosaminoglykane Mischpolymere (Integra LifeScience)
6) Fibrin (Beriplast®, Aventis; Tissucol®, Baxter)
7) Konservierte humane Spalthaut (Leichenhaut)

Als Nachteile der Matrizes 1 - 4 zeigen sich bei ihrer Verwendung als Komposite mit humanen Zellen in den meisten Fällen für die Zellen nachteilige Wirkungen durch die beim Abbau der Matrizes entstehenden Metabolite (z.B. freies Lactat, Hyaluronsäure- oder Polyurethanmonomere). Das entstehende Ersatzgewebe wird auch als mechanisch und plastisch-chirurgisch unbefriedigend beschrieben. Die Verwendung von Dextran beinhaltet die Gefahr der Induktion allergischer Reaktionen oder - insbesondere im Falle von Beads -, deren Enzystierung. (5.) Kollagenderivate oder Kollagen-Glykosaminoglykane Mischpolymere werden dagegen ohne die Bildung nachteiliger Produkte abgebaut und sind sowohl mechanisch als auch plastisch-chirurgisch von deutlich höherer Qualität. Die positiven Eigenschaften dieser Matrizes sind vielfach publiziert. Die bei der Verwendung von Kollagenderivaten und auch bei Leichenhaut verwendeten Schichtdicken müssen jedoch kritisch diskutiert werden. Dicke Schichten können der für eine schnelle Wundheilung dringend erforderlichen, schnellen Vaskularisierung und Rezellulierung hinderlich sein. Insbesondere bei Kombination dieser Materialien mit epithelialen Zellen kann die Schichtdicke potentiell der limitierende Faktor sein, da sich das Epithel naturgemäß auf der dem Nährstoffstrom abgewandten Seite befindet.

(6.) Fibrin stellt eine neue Möglichkeit einer biokompatiblen Matrix dar. Fibrin ist als chirurgischer Zwei-Komponenten-Wundkleber (Zulassung als Arzneimittel) kommerziell erhältlich. Es wird aus getesteten humanen Plasmapools gewonnen. Fibrin kann in beliebig dünnen Schichten ausgebracht werden, ist allerdings für ein Handling als Schicht zu fragil. Fibrin kann als Unterlage für die Kultur von Zellen verwendet werden und hat keine negativen Effekte auf die vitalen und proliferativen Eigenschaften von Zellkulturen (PELLEGRINI et al.: The control of epidermal stem cells (holoclones) in the treatment of massive full-thickness bums with autologous keratinocytes cultured on fibrin. Transplantation, 6, 868-879, (1998)).

Als Trägermatrix für suspendierte Epithelzellen wird Fibrin bereits eingesetzt. Die Methode verwendet enzymatisch vereinzelte Epithelzellen aus der Zellkultur, die zunächst in einer der Kleberkomponenten suspendiert werden. Während des Polymerisierungsprozesses werden sie vollständig in den Kleber eingeschlossen.

(7.) Allogene humane Leichenhaut ist als Behandlungsstandard in der Verbrennungsmedizin etabliert. Durch stetige Verbesserung der Entnahmetechnik ist es möglich, aus dem Spendermaterial sehr dünne Spalthaut zu gewinnen. Das Material, das vorwiegend aus extrazellulärer Matrix und nur zu einem sehr geringen Anteil aus zellulären Anteilen besteht, wird vom Körper angenommen und bis zu ihrer Abstoßung integriert. Bei sachgerechter Präparation bleiben wichtige Bestandteile (z.B. Basalmembranen, Hohlräume der Vaskularisierung) erhalten. Damit ist dieses Material für eine Kombination mit der Zellkulturtechnik geeignet. Für die Schichtdicke des Materials gelten allerdings die gleichen Einschränkungen wie für artifizielle Matrizes (siehe (5)).

Es besteht daher weiterhin ein großes Bedürfnis nach Hautmatrizes, welche eine stabile, rasch anheilende und vaskularisierende Abdeckung und Regenerierung verletzter Hautpartien ermöglichen. Die Erfinder haben sich daher die Aufgabe gestellt solche Matrizes und Verfahren zu ihrer Herstellung zu finden.

Die Lösung dieser Aufgabe ist durch die Merkmale der unabhängigen Ansprüche gegeben und wird durch die Merkmale der Unteransprüche gefördert.

### Beschreibung des Verfahrens

Die Verwendung von Fibrin in Verbindung mit gemeshter und weitlumig expandierter Leichenhaut oder artifiziellen biodegradierbaren Hautersatzwerkstoffen vereint die Vorteile stabiler und permanent verbleibender biologischer Matrizes mit der Möglichkeit Fibrin als dünnen biokompatiblen, zellkulturgeeigneten und biodegradierbaren Träger für vitale Zellkulturen zu verwenden.

### Etablierung autologer und allogener Keratinozytenkulturen

Die Zellkulturen werden nach dem standardisierten Protokoll von Rheinwald und Green zunächst aus einer Hautbiopsie isoliert und in einer Primärkultur auf Feederlayern etabliert und vermehrt.

Dazu werden aus jeweils einem Quadratzentimeter Haut ca. 1,5-3x10⁶ Keratinozyten isoliert und für die Primärkultur in einer Dichte von 1 - 7x10⁶ auf einem Feederlayer proliferationsinhibierter Fibroblasten ausgesät.
Aus der gleichen Biopsie können bei weitergehenden Fragestellungen auch die anderen in einer Hautbiopsie präsenten Zelltypen (z.B. Fibroblasten, Endothelzellen, Melanozyten) durch fraktionierte Isolation gewonnen und in Zellkultur etabliert werden.
Die Techniken ermöglichen die Etablierung sowohl autologer als auch allogener Kulturen.

### Matrixpräparation

1. Humane Leichenhaut (nativ oder glyzerinkonserviert oder kryokonserviert oder dezelluliert, mit und ohne Restvitalität) oder artifizielle Matrixmaterialien werden wie zu einer Transplantation vorbereitet (Rehydratation, auswaschen in physiologischen Medien), dann aber mit einem zur Expansion von Spalthaut klinisch verwendeten Meshgerät (z.B. Zimmer, Brennen, Aeskulap) netzartig eingeschnitten (gemesht) und im Verhältnis 1:1,5 - 1:8 auf die gewünschte Maschengröße expandiert in eine Zellkulturschale ausgebracht. Alternativ kann zur Gewebeexpansion die MEEK-Technik (Brandt: Chirurgische Strategien bei der Behandlung Brandverletzter, Chirurg 66 (1995) 243-250) verwendet werden.
2. Mit den gleichen Techniken können artifizielle Hautersatzmaterialien (Integra®, Capronolactonnetze, Polyglactinnetze (Vicryl®), Kollagenschwämme) für eine gleichartige Verwendung vorbereitet werden.

Die verwendeten Matrixmaterialien weisen vorzugsweise eine Dicke von 0,05-0,5 mm auf, wobei das Netz eine Zwischenraumfläche von 20-90%, vorzugsweise 60-80 % der Gesamtfläche aufweist.

### Präparation des Fibrinträgerfilms

Die erfindungsgemässe Fibrinmatrix kann aus Fibrinschichten aus bekannten Fibrinklebern mittels Koagulations-mitteln Thrombin, Faktor XIII und Aprotinin hergestellt werden. Die Fibrinschicht kann weiterhin Wachstumsstimulanzien enthalten.

Die Komponenten des Fibrinklebers werden nach Vorschrift des Herstellers verwendet oder in Abänderung des Herstellungsvorschriften mit geeigneten Salzlösungen (z.B. NaCl 1,1% mit CaCl₂ 1mM) verdünnt. Die Fibrinogenkomponente ist für den hier beschriebenen Zweck unverdünnt oder bis zu einem Verdünnungsverhältnis von 1:10 verwendbar.

Die Thrombinkomponente wird bei der Herstellung einer zellfreien Unterlage unverdünnt (500 - 1500 IE) verwendet oder bei einer gewünschten Verzögerung der Polymerisierung und/oder bei der Integration von Zellen in einem Bereich von 1IE bis 500IE mit geeigneten Salzlösungen (z.B. NaCl 1,1% mit CaCl₂ 1mM) verdünnt.

Die Mischung der Komponenten erfolgt unmittelbar vor dem Ausbringen in die Interstitien des vorbereiteten Matrixmaterials. Die Ausbringung erfolgt durch Gießen (casting) oder durch Sprühen mit einem geeigneten Druckzerstäuber (z.B. Tissomat, Fa. Baxter). Das Komposit wird dann als Unterlage für die Keratinozytenzellkulturen verwendet.

Weiterhin ist es möglich weitere aus dem Patientenmaterial isolierte und vermehrte Zellen (z.B. autologe Fibroblasten und/oder Endothelzellen und/oder Melanozyten) in das Fibrin zu integrieren. Dazu werden die Zellen nach ihrer Isolation aus dem Spendergewebe in Kultur etabliert und vermehrt, um aus dem Gesamtisolat adhäsionsfähige und teilungsaktive Zellen zu selektierten. Zellen mit geringer Ausprägung dieser Eigenschaften werden durch die Kulturbedingung ausgesondert. Erst nach diesem Schritt werden die Zellen mit der Matrix kombiniert. Dies geschieht durch das Suspendieren der Zellen in der Thrombinkomponente des Klebers. Die gewünschten Zelltypen können gleichzeitig einpolymerisiert oder bei der Verwendung der Sprühtechnik in verschiedenen Lagen ausgebracht werden. Dieses Vorgehen erlaubt es die Zellen nach ihrer spezifischen Herkunft anzuordnen oder in ihrer physiologischen Orientierung auf die Matrix aufzubringen.

### Vorteile der neuartigen Matrix sind die

- ausschließliche Verwendung physiologischer Ausgangsmaterialien
- vollständige Integration (Zellen, Dermis, artifizielle Matrixmaterialien) oder vollständiger Abbau (Fibrin) der Kompositbestandteile
- ein stabiles Dermis- oder Dermisersatzmesh wird kombiniert mit einem sehr dünnen Zellträger (Fibrin) der eine schnelle Anbindung der Epithelzellen an den Nährstoffstrom gewährleistet.
- Das Konzept bietet durch die Kombination verschiedener Zellen die Möglichkeit eine deutliche Verbesserung der Heilung großflächiger Wunden zu erreichen
- Durch die Verwendung azellulärer Dermisersatzmaterialien werden unerwünschte Immunreaktionen auf verbliebene allogene Zellbestandteile vermieden
- Verwendete Zellen können in physiologisch richtiger Weise auf (Keratinozyten, Melanozyten) oder in (Fibroblasten, Endothelzellen) eine Matrix gebracht werden
- Der Aufbau der Matrix erlaubt es die gewünschten Zelltypen gleichzeitig oder nacheinander, sowie sequentiell geschichtet auszubringen.
- Die Fibrinmatrix ermöglicht die Integration zellwachstumsfördernder Substanzen in die transplantierbare Matrix.

## Patentansprüche

1. Hautmatrix zur Abdeckung und Regenerierung verletzter Hautpartien enthaltend
a) ein Netz aus gemeshter oder nach der MEEK-Technik aufgearbeiteter und weitlumig expandierter autologer oder allogener humaner Haut, in nativer, avitalisierter, dezellulierter oder anderweitig bearbeiteter Form oder nach der MEEK-Technik aufgearbeiteter und weitlumig expandierter artifizieller Hautersatzmaterialien.
b) wobei in den Zwischenräumen des Netzes eine Fibrinschicht angeordnet ist, die
c) auf ihrer Oberseite Keratinozytenzellen und/oder in ihrer Masse Fibroblasten und/oder Endothelzellen und/oder Melanoyztenzellen enthält.

2. Hautmatrix, gemäß Anspruch 1, **dadurch gekennzeichnet, dass** die Zellen adhäsionsfähige, teilungsaktive Zellen sind, die aus Zellkulturen von Hautzellen des Patienten stammen.

3. Hautmatrix gemäß Anspruch 1 oder 2, **dadurch gekennzeichnet, dass** verschiedene Zelltypen in übereinandergeschichteten Lagen der Fibrinschicht enthalten sind.

4. Hautmatrix gemäß einem der Ansprüche 1-3, **dadurch gekennzeichnet, dass** die Fibrinschicht Wachstumsstimulanzien enthält.

5. Hautmatrix gemäß einem der Ansprüche 1-4, **dadurch gekennzeichnet, dass** das Netz a) aus Konservierter humaner Spalthaut in dezellulierter Form besteht.

6. Hautmatrix gemäß einem der Ansprüche 1-4, **dadurch gekennzeichnet, dass** das Netz a) aus artifiziellen Hautersatzmaterialien besteht.

7. Hautmatrix gemäß einem der Ansprüche 1-6, **dadurch gekennzeichnet, dass** die Fibrinschicht aus bekannten Fibrinklebern mittels Koagulationsmitteln Thrombin, Faktor XIII und Aprotinin hergestellt wird.

8. Hautmatrix gemäß einem der Ansprüche 1-7, **dadurch gekennzeichnet, dass** die Matrix eine Dicke von 0,05-0,5 mm aufweist.

9. Hautmatrix gemäß einem der Ansprüche 1-8, **dadurch gekennzeichnet, dass** das Netz a), eine Zwischenraumfläche von 20-90%, vorzugsweise 60-80 % der Gesamtfläche aufweist.

10. Verfahren zur Herstellung von Hautmatrix gemäß einem der Ansprüche 1-9, **dadurch gekennzeichnet, dass** man
a) Hautzellen des Patienten entnimmt und in bekannter Art und Weise auf geeigneten Kulturmedien vermehrt und nach Zelltypen selektiert,
b) Matrixmaterial aus autologer oder allogener humaner Haut, in nativer, avitalisierter, dezellulierter oder anderweitig bearbeiteter Form oder artifiziellen Hautersatzmaterialien im Verhältnis 1:1,5-1:8 mesht oder nach der MEEK-Technik weitlumig expandiert, wodurch ein Netz gebildet wird,
c) Fibrinkleber in wässriger Lösung mit den Koagulationsmitteln mischt, Zellkulturmaterial und ggf.wachstumsstimulierende Substanzen zufügt und in die Zwischenräume des Netzes nach b) in einer oder mehreren Lagen einbringt und verfestigt,

11. Verfahren gemäß Anspruch 10, entsprechend **dadurch gekennzeichnet, dass** mehrere Lagen Fibrinkleber mit unterschiedlichen Zelltypen eingebracht werden.

12. Verfahren gemäß Anspruch 10 oder 11, **dadurch gekennzeichnet, dass** auf die Oberseite der Matrix Keratinozytenzellen aufgebracht und kultiviert werden.

## Claims

1. Skin matrix for the covering and regeneration of injured skin parts containing
a) a network of meshed or allegeneic human skin or autologous or allogeneic human skin worked up according to the MEEK technique and wide-lumen expanded, in native, avitalised, decellulated or otherwise worked form or artificial skin replacement materials worked up according to the MEEK technique and wide-lumen expanded,
b) whereby a fibrin layer is arranged in the intermediate spaces of the network which
c) on its upper side contains keratinocyte cells and/or in its mass contains fibroblasts and/or endothelial cells and/or melanocyte cells.

2. Skin matrix according to claim 1, **characterised in that** the cells are adhesive, division-active cells which originate from cell cultures of skin cells of the patients.

3. Skin matrix according to claim 1 or 2, **characterised in that** there are contained different cell types in overlying layers of the fibrin layer.

4. Skin matrix according to one of claims 1 - 3, **characterised in that** the fibrin layer contains growth stimulants.

5. Skin matrix according to one of claims 1 - 4, **characterised in that** the network a) consists of preserved human split skin in decellulated form.

6. Skin matrix according to one of claims 1 to 4, **characterised in that** the network a) consists of artificial skin replacement materials.

7. Skin matrix according to one of claims 1 - 6, **characterised in that** the fibrin layer is produced from known fibrin adhesives by means of coagulation agents thrombin, Factor XIII and aprotinin.

8. Skin matrix according to one of claims 1 - 7, **characterised in that** the matrix has a thickness of 0.05 - 0.5 mm.

9. Skin matrix according to one of claims 1 - 8, **characterised in that** the network a) has an intermediate surface area of 20 - 90%, preferably 60 - 80% of the whole surface.

10. Process for the production of skin matrix according to one of claims 1 - 9, **characterised in that** one
a) removes skin cells of the patient and propagates in known manner on suitable culture media and selects according to cell types,
b) meshes matrix material from autologous or allogeneic human skin in native, avitalised, decellulated or otherwise worked up form in the ratio of 1:1.5 - 1:8 or wide-lumen expands according to the MEEK technique, whereby a network is formed,
c) mixes fibrin adhesive in aqueous solution with the coagulation agents, adds thereto cell culture material and possibly growth-stimulating substances and, into the intermediate spaces of the mesh according to b), introduces one or more layers and solidifies.

11. Process according to claim 10, correspondingly **characterised in that** several layers of fibrin adhesive with differing cell types are introduced.

12. Process according to claim 10 or 11, **characterised in that**, on the upper side of the matrix, keratinocyte cells are applied and cultured.

## Revendications

1. Matrice de peau pour la couverture et régénération de parties de peau lésées comprenant
a) un réseau de peau humaine maillée ou remise en état selon la technique MEEK et à large lumen, expansée, autologue ou allogénique, sous forme native, dévitalisée, décellularisée ou traitée par d'autres moyens, ou des matières artificielles de substitution de la peau, expansées, à large lumen et traitées selon la technique MEEK.
b) une couche de fibrine étant disposée dans les interstices du réseau, laquelle
c) contient sur sa face supérieure des cellules kératinocytaires et/ou des fibroblastes et/ou des cellules endothéliales et/ou des cellules mélanocytaires dans sa masse.

2. Matrice de peau, selon la revendication 1, **caractérisée en ce que** les cellules sont des cellules capables d'adhérer, en division active, provenant de cultures cellulaires de cellules cutanées du patient.

3. Matrice de peau selon la revendication 1 ou 2, **caractérisée en ce que** différents types de cellules sont contenus dans des couches superposées de la couche de fibrine.

4. Matrice de peau selon l'une des revendications 1 à 3, **caractérisée en ce que** la couche de fibrine contient des stimulateurs de croissance.

5. Matrice de peau selon l'une des revendications 1 à 4, **caractérisée en ce que** le réseau a) est constitué de peau humaine fendue conservée, sous forme décellularisée.

6. Matrice de peau selon l'une des revendications 1 à 4, **caractérisée en ce que** le réseau a) est constitué de matières artificielles de substitution de la peau.

7. Matrice de peau selon l'une des revendications 1 à 6, **caractérisée en ce que** la couche de fibrine est fabriquée à partir de glutens de fibrine connus au moyen des coagulants tels que la thrombine, le facteur XIII et l'aprotinine.

8. Matrice de peau selon l'une des revendications 1 à 7, **caractérisée en ce que** la matrice présente une épaisseur de 0,05 à 0,5 mm.

9. Matrice de peau selon l'une des revendications 1 à 8, **caractérisée en ce que** le réseau a) présente une surface interstitielle de 20 à 90 %, de préférence de 60 à 80 % de la surface totale.

10. Procédé de fabrication de matrice de peau selon l'une des revendications 1 à 9, **caractérisé en ce que**
a) on prélève des cellules cutanées du patient et les multiplie sur des milieux de culture appropriés de manière connue et les sélectionne par types de cellules,
b) on maille de la matière matricielle en peau humaine autologue ou allogénique, sous forme native, dévitalisée, décellularisée ou traitée par d'autres moyens, ou des matières artificielles de substitution de la peau dans le rapport 1 :1,5-1 :8, ou on les expanse avec un large lumen selon la technique MEEK, en formant ainsi un réseau,
c) on mélange du gluten de fibrine en solution aqueuse avec les coagulants, on ajoute de la matière de culture cellulaire et, le cas échéant, des substances stimulatrices de croissance et on les introduit et les consolide dans les interstices du réseau selon b) en une ou plusieurs couches.

11. Procédé selon la revendication 10, **caractérisé en ce que** plusieurs couches de gluten de fibrine sont introduites avec différents types de cellules.

12. Procédé selon les revendications 10 ou 11, **caractérisé en ce que** des cellules kératinocytaires sont déposées et cultivées à la face supérieure de la matrice.
